(19) Europäisches Patentamt
European Patent Office
Office européen des brevets

(11) **EP 0 695 202 B1**

(12) **FASCICULE DE BREVET EUROPEEN**

(45) Date de publication et mention
de la délivrance du brevet:
**23.08.2000 Bulletin 2000/34**

(21) Numéro de dépôt: **93910118.4**

(22) Date de dépôt: **13.05.1993**

(51) Int. Cl.⁷: **A61M 25/01**

(86) Numéro de dépôt international:
**PCT/FR93/00465**

(87) Numéro de publication internationale:
**WO 93/23109 (25.11.1993 Gazette 1993/28)**

(54) **INSTRUMENT CHIRURGICAL POUR OPERATION D'ANESTHESIE PERIDURALE**

CHIRURGISCHES INSTRUMENT ZUR PERIDURALANÄSTHIE

SURGICAL INSTRUMENT FOR EPIDURAL ANESTHESIA

(84) Etats contractants désignés:
**AT BE CH DE DK ES FR GB GR IE IT LI LU MC NL PT SE**

(30) Priorité: **14.05.1992 FR 9205857**

(43) Date de publication de la demande:
**07.02.1996 Bulletin 1996/06**

(73) Titulaire: **Société VYGON**
**F-95440 Ecouen (FR)**

(72) Inventeur: **BRINON, Thierry**
**F-95560 Montsoult (FR)**

(74) Mandataire:
**Martin, Jean-Jacques et al**
**Cabinet REGIMBEAU**
**26, Avenue Kléber**
**75116 Paris (FR)**

(56) Documents cités:
**EP-A- 0 406 586          WO-A-90/10466**
**CH-A- 288 589           US-A- 4 500 313**
**US-A- 5 084 022**

**Description**

La présente invention concerne le domaine des instruments chirurgicaux comprenant une aiguille et un cathéter associé, susceptible de coulisser dans l'aiguille, notamment pour opération d'anesthésie péridurale.

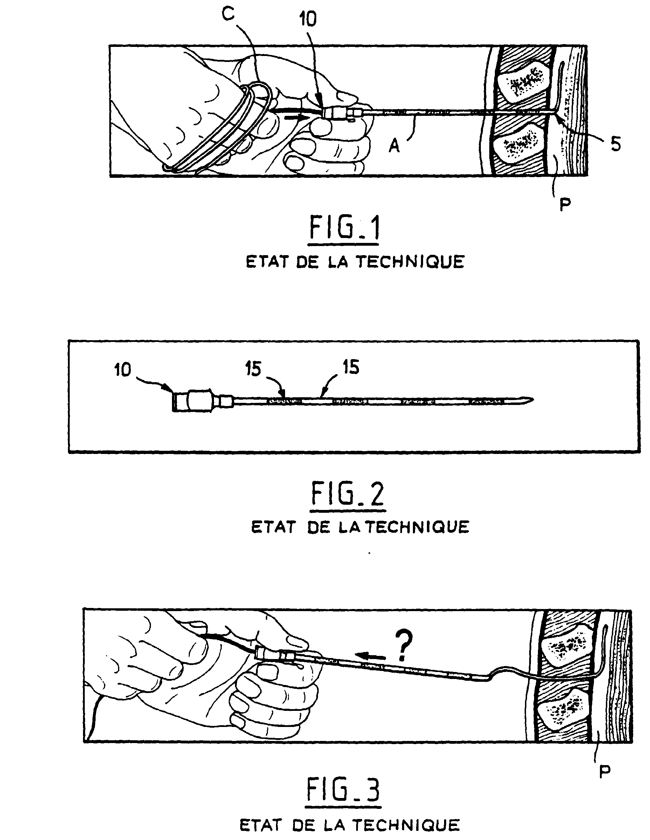

L'anesthésie par voie péridurale est une technique largement pratiquée de nos jours, et consiste, comme illustré sur la figure 1, à mettre en place un cathéter C dans l'espace péridural P d'un patient pour y injecter un anesthésique, à l'aide d'une aiguille A munie à son extrémité distale 5 d'une pointe effilée en biseau, encore appelée aiguille Tuohy.

Pour effectuer une opération d'anesthésie péridurale, l'anesthésiste commence généralement par insérer l'aiguille A dans le dos du patient jusqu'à ce que son extrémité distale 5 débouche dans l'espace péridural P, puis le cathéter C est, introduit par l'ouverture proximale 10 de l'aiguille, jusqu'à déboucher dans l'espace péridural P par l'extrémité distale 5 de celle-ci.

Il est important, afin d'éviter de traumatiser cette région sensible du patient, de ne pas enfoncer trop profondément l'aiguille, donc de connaître la longueur d'aiguille introduite dans le dos du patient. Pour ce faire, une aiguille Tuohy A conforme à l'art antérieur est généralement munie, comme représenté sur la figure 2, de marques 15 axialement équiréparties sur sa longueur, de sorte que l'anesthésiste peut déduire par calcul la longueur d'aiguille introduite dans le dos du patient, en connaissant le nombre total de marques que comporte l'aiguille et en soustrayant à ce dernier le nombre de marques restant apparentes après introduction de l'aiguille.

Il est également important de connaître la longueur de cathéter introduite dans l'espace péridural, et le cathéter C est généralement muni à cet effet de marques identiques axialement équiréparties sur sa longueur. Pour connaître la longueur de cathéter s'étendant dans l'espace péridural P, l'anesthésiste procède généralement en comptant le nombre de marques portées par le cathéter C franchissant l'ouverture proximale 10 de l'aiguille lorsque le cathéter est poussé dans l'aiguille, après sa sortie par le biseau de celle-ci, ce que l'anesthésiste peut aisément détecter, puisque l'introduction du cathéter dans l'aiguille se fait plus difficilement dès lors que ce dernier s'étend hors de l'extrémité distale de celle-ci. Ensuite, l'aiguille Tuohy A est retirée tandis que le cathéter est laissé en place dans l'espace péridural, pour y injecter une solution d'anesthésique.

L'anesthésiste rencontre plusieurs difficultés survenant au cours des différentes étapes d'une opération d'anesthésie péridurale telle qu'elle vient d'être décrite.

Tout d'abord, pour connaître les longueurs d'aiguille ou de cathéter introduites dans le dos du patient, le chirurgien doit noter ou retenir les nombres totaux de graduations portées par l'aiguille et le cathéter et déduire de ceux-ci les nombres de graduations restant apparentes après l'introduction de l'aiguille ou du cathéter, ce qui est malaisé dans le contexte d'une intervention chirurgicale.

Par ailleurs, lors de l'extraction de l'aiguille, le cathéter tend à être entraîné avec celle-ci par frottement, et il est alors difficile de connaître avec précision la longueur de cathéter restant dans l'espace péridural après retrait de l'aiguille, problème qui, est schématiquement illustré sur la figure 3.

Enfin, le cathéter pouvant rester en place pendant plusieurs heures, celui-ci risque de se déplacer durant cette période et il est important de connaître sa position avec précision avant toute nouvelle injection d'anesthésique.

On a décrit dans la publication WO 90/10466 un instrument chirurgical comprenant une aiguille destinée à l'introduction d'un cathéter, notamment pour une opération d'anesthésie péridurale. Le cathéter est constitué d'une partie proximale rigide et d'une partie distale souple. Des marques sont disposées sur le cathéter pour repérer visuellement la jonction des parties souple et rigide, de manière à éviter d'engager la partie rigide dans l'espace péridural, pour ne pas causer de traumatisme au patient.

On a décrit dans la demande EP-0 406 586 un instrument chirurgical comprenant une aiguille munie de marques axialement équiréparties sur sa longueur.

La présente invention a pour objet un instrument chirurgical amélioré, comprenant une aiguille et un cathéter associé, susceptible de coulisser dans l'aiguille, notamment pour opération d'anesthésie péridurale, remédiant aux principales difficultés précédemment citées.

Cet instrument chirurgical est défini dans les revendications 1 et suivantes.

D'autres caractéristiques et avantages de l'invention apparaîtront à la lecture détaillée qui va suivre, d'un exemple de réalisation non limitatif de la présente invention, et à l'examen du dessin annexé sur lequel :

- la figure 1, précédemment décrite, montre l'insertion d'un cathéter dans l'espace péridural d'un patient,
- la figure 2 montre une aiguille munie de marques équiréparties axialement, précédemment décrite, illustrant l'état de la technique,
- la figure 3, précédemment décrite, illustre le problème posé dans l'art antérieur par le retrait de l'aiguille du dos du patient en laissant en place le cathéter dans l'espace péridural,
- la figure 4 montre en vue de face une aiguille partie d'un instrument chirurgical conforme à l'invention,
- la figure 5 est une vue de côté, prise dans le plan de la vue de face de la figure 4 d'une aiguille et de son cathéter associé, conforme à l'invention,

- la figure 6 est une vue de dessous du cathéter représenté sur la figure 5,
- les figures 7 à 11 illustrent les différentes étapes d'une opération d'anesthésie péridurale, effectuée à l'aide d'un instrument chirurgical conforme à l'invention,
- la figure 12 montre comment s'effectue le calcul de la longueur de cathéter débouchant dans l'espace péridural après retrait de l'aiguille.

Les figures 1 à 3 ont été précédemment décrites pour illustrer l'état de la technique.

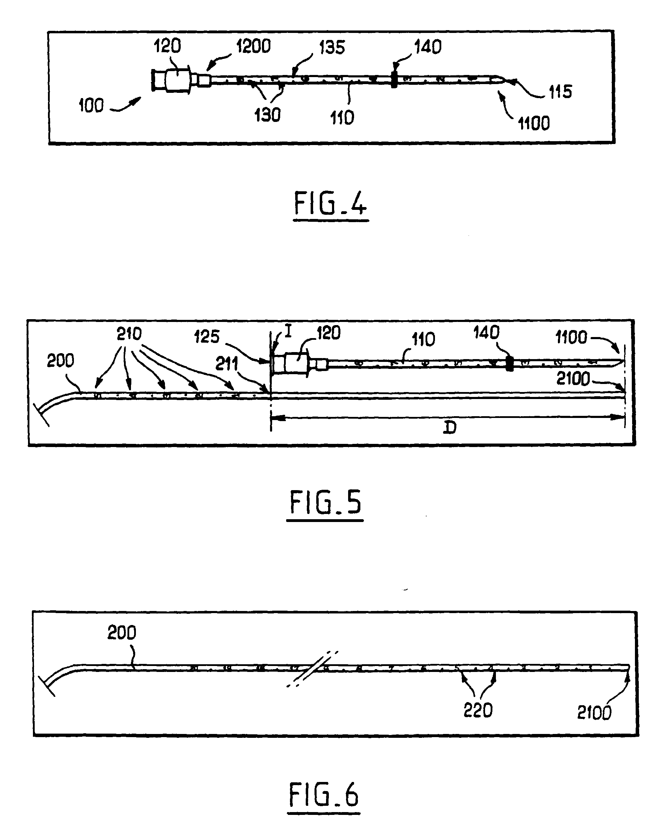

On aperçoit sur la figure 4 une aiguille référencée dans son ensemble 100 comprenant un corps d'aiguille 110 creux, propre à être introduit par son extrémité distale 1100 dans le dos d'un patient, et muni à l'autre extrémité 1200 d'un embout de raccordement 120. Comme représenté, l'extrémité distale 1100 est de préférence effilée pour former un biseau 115.

Conformément à une caractéristique de l'invention, l'aiguille 100 est munie sur son corps 110 de graduations 130 axialement réparties sur celui-ci, propres à indiquer directement, par simple lecture, sans calculs, la longueur d'aiguille s'étendant depuis l'extrémité distale 1100 jusqu'à chacune desdites graduations 130.

De préférence, comme représenté, ces graduations 130 comportent des chiffres arabes imprimés ou gravés tous les centimètres depuis l'extrémité distale 1100, et des points 135 placés à mi-distance de chacune desdites graduations 130.

Conformément à une autre caractéristique de l'invention, il est prévu un curseur 140 monté coulissant sur le corps 110 de l'aiguille, propre à prendre appui sur un bord d'une incision pratiquée sur le corps du patient, par où l'aiguille est introduite, pour se déplacer sur le corps 110 d'une distance correspondant à la longueur d'aiguille introduite par ladite incision, comme cela sera précisé dans la suite.

On a représenté sur la figure 5 un instrument chirurgical conforme à la présente invention, comprenant l'aiguille 100 précédemment décrite en référence à la figure 4 et un cathéter associé référencé 200, susceptible de coulisser dans le corps 110 et l'embout 120, et propre à être introduit par une ouverture 125 prévue sur l'embout de raccordement 120.

Conformément à une caractéristique de l'invention, le cathéter 200 est muni d'un ensemble de repères axiaux principaux référencés 210, susceptibles de coïncider, lorsque le cathéter 200 est introduit dans l'aiguille 100, avec un index I défini sur l'aiguille, pour indiquer, par simple lecture, sans calculs, la longueur de cathéter s'étendant hors de l'extrémité distale 1100 de l'aiguille.

On peut proposer de positionner l'index I à divers endroits de l'aiguille 100 sans sortir du cadre de la présente invention. De préférence, le plan de l'ouverture 125 de l'embout de raccordement sert d'index I, mais, bien entendu, on peut proposer de ménager une fenêtre dans le corps 110 de l'aiguille 100, munie éventuellement d'une loupe de grossissement ou d'un autre dispositif susceptible de faciliter la lecture des repères du cathéter.

De préférence, conformément à une caractéristique avantageuse de l'invention, cet ensemble de repères axiaux principaux 210 comprend des graduations principales régulièrement espacées, s'étendant à partir d'une origine 211 située à une distance D de l'extrémité distale 2100 du cathéter, correspondant à la distance entre l'extrémité distale 1100 de l'aiguille 100 et l'index I défini sur celle-ci. Comme représenté sur la figure 5, ces graduations principales sont espacées d'un centimètre entre elles et comprennent de préférence des chiffres arabes disposés, par gravure ou impression, tous les centimètres depuis l'origine 211, séparés par un trait ou un point, disposé à mi-distance.

Le cathéter 200 est muni d'un ensemble de repères axiaux auxiliaires 220, aisément différenciables des repères principaux 210, propres à indiquer par simple lecture, sans calculs, la longueur de cathéter s'étendant depuis son extrémité distale 2100 jusqu'à chacun desdits repères auxiliaires 220, comme cela est représenté sur la figure 6. De préférence, cet ensemble de repères auxiliaires 220 comprend des graduations auxiliaires régulièrement espacées s'étendant à partir d'une origine coïncidant avec l'extrémité distale 2100 du cathéter. De préférence également, ces graduations auxiliaires 220 sont des chiffres arabes espacés d'un centimètre entre eux, séparés par un trait ou un point placé à mi-distance entre deux chiffres. Avantageusement, les repères principaux et auxiliaires sont disposés sur le cathéter pour former deux échelles au voisinage l'une de l'autre, en vue de faciliter la lecture. En variante, les repères principaux et auxiliaires peuvent être disposés sur des faces diamétralement opposées du cathéter 200. Les repères principaux 210 et auxiliaires 220 sont avantageusement de couleurs différentes.

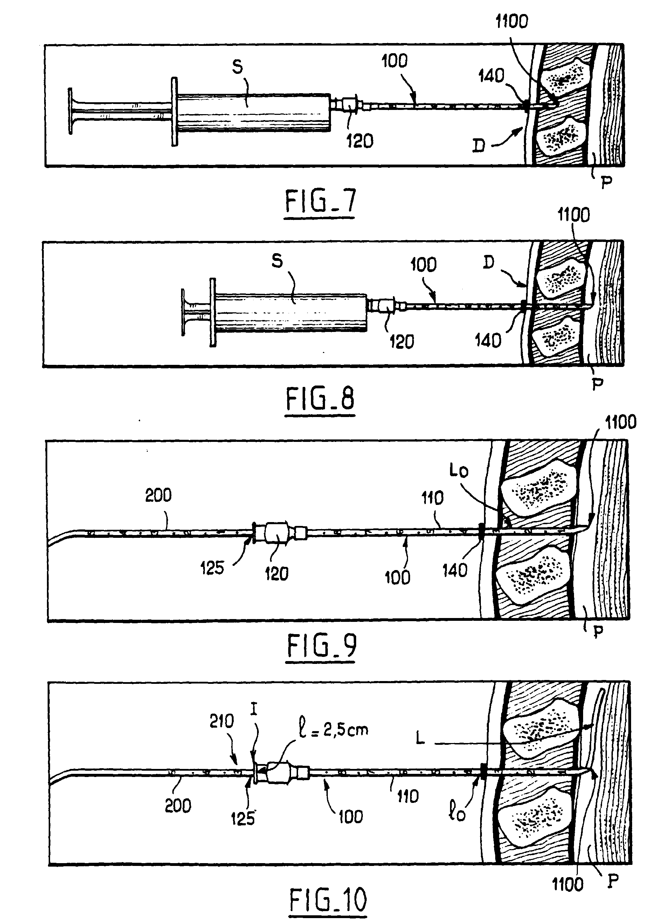

Les différentes étapes de l'utilisation d'un instrument chirurgical conforme à la présente invention vont être décrites en référence aux figures 7 à 11.

On commence par effectuer une anesthésie de l'espace interépineux, selon des techniques connues en elles-mêmes, puis l'aiguille 100, munie de son curseur coulissant 140 préalablement positionné au niveau de l'extrémité distale 1100, est introduite dans le dos D du patient, l'aiguille 100 étant en outre raccordée par l'embout 120, de façon étanche, à une seringue S, pour constituer ce que l'homme de l'art appelle un mandrin gazeux ou liquide, selon que la seringue 5 est remplie d'un gaz ou d'un liquide. Cette technique à mandrin gazeux ou liquide permet à l'anesthésiste de repérer précisément lorsque l'extrémité distale 1100 de l'aiguille débouche dans l'espace péridural P. Habituellement, l'anesthésiste pousse d'une main l'aiguille 100 en direction de l'espace péridural P, tandis que son autre main comprime le mandrin gazeux ou liquide jusqu'à son effondrement, qui a lieu lorsque l'aiguille 100 débouche dans l'espace péridural P, comme cela est représenté sur les figures 7 et 8. On remarque également à l'examen de ces figures que le curseur 140 s'est déplacé sur l'aiguille 100 d'une distance $L_0$ correspondant à la longueur d'aiguille introduite dans le corps du patient.

Ensuite, conformément à la figure 9, la seringue S est déconnectée, et le cathéter 200 est introduit dans l'aiguille par l'ouverture 125 de l'embout 120, qui sert également d'index I.

Le cathéter est poussé dans l'aiguille, jusqu'au niveau de l'extrémité distale 1100.

L'anesthésiste peut alors à tout moment, sans calculs, connaître la longueur L de cathéter introduite dans l'espace péridural P en lisant quelle est la valeur 1 de la graduation principale 210 du cathéter qui coïncide sensiblement avec l'index 125, comme cela est illustré sur la figure 10.

Dans l'exemple de la figure 10, on lit L = 2,5 cm puisque l'index I se trouve entre les graduations 2 et 3.

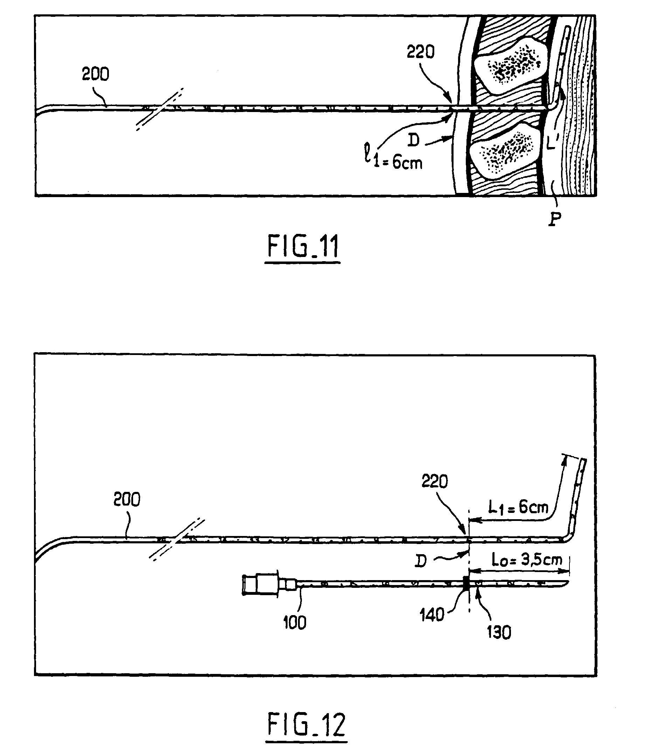

Après retrait de l'aiguille 100, le chirurgien peut, par simple lecture des repères auxiliaires 220 du cathéter 200, savoir, sans calculs, quelle est la longueur $L_1$ du cathéter s'étendant à l'intérieur du corps du patient, puis en déduire aisément la longueur L' du cathéter 200 s'étendant à l'intérieur de l'espace péridural P, en retranchant à la longueur $L_1$ donnée par la valeur $l_1$ de la graduation auxiliaire 220 la plus proche du corps du patient, la longueur $L_0$, donnée par la valeur $l_0$ de la graduation 130 d'aiguille 100 indiquée par le curseur 140, comme illustré sur les figures 11 et 12. Dans l'exemple des figures 10, 11 et 12 le retrait de l'aiguille s'est effectué sans entraînement du cathéter hors de l'espace péridural puisque L = 2,5 cm et L' = $L_1 - L_0$ = 6 - 3,5 = 2,5 cm = L .

Finalement, un instrument chirurgical selon l'invention s'avère particulièrement simple et rapide à utiliser et peut être appliqué, sans sortir du cadre de l'invention, à des opérations chirurgicales autres que l'anesthésie péridurale, par exemple d'autres anesthésies loco-régionales.

## Revendications

1. Instrument chirurgical comprenant une aiguille (100) et un cathéter (200) associé, susceptible de coulisser dans l'aiguille, notamment pour opération d'anesthésie péridurale, l'aiguille étant munie de graduations (130) axialement réparties, propres à indiquer directement, par simple lecture, sans calculs, la longueur d'aiguille s'étendant depuis son extrémité distale (1100) jusqu'à chacune desdites graduations (130). caractérisé en ce que l'aiguille est munie d'un curseur (140) monté coulissant sur l'aiguille (100), propre à prendre appui sur un bord d'une incision par où l'aiguille est introduite, et adapté pour se déplacer sur celle-ci d'une distance correspondant à la longueur d'aiguille introduite dans ladite incision, et en ce que le cathéter est muni d'un ensemble de repères axiaux principaux (210) susceptibles de coïncider, lorsque le cathéter (200) est introduit dans l'aiguille (100), avec un index (I) défini sur celle-ci, pour indiquer, par simple lecture, sans calculs, la longueur (L) de cathéter s'étendant hors de l'extrémité distale (1100) de l'aiguille, et est muni d'un ensemble de repères axiaux auxiliaires (220) aisément différenciables des repères principaux (210), propres à indiquer, par simple lecture, sans calculs, la longueur de cathéter s'étendant depuis son extrémité distale (2100) jusqu'à chacun desdits repères auxiliaires (220).

2. Instrument chirurgical selon la revendication 1, caractérisé en ce que ledit ensemble de repères axiaux principaux (210) comprend des graduations principales régulièrement espacées s'étendant à partir d'une origine (211) située à une distance (D) de l'extrémité distale (2100) du cathéter correspondant à la distance entre l'extrémité distale de l'aiguille (1100) et l'index (I) défini sur celle-ci.

3. Instrument chirurgical selon la revendication 2, caractérisé en ce que lesdites graduations principales (210) sont espacées d'un centimètre entre elles.

4. Instrument chirurgical selon l'une des revendications 1 à 3, caractérisé on ce que ledit ensemble de repères auxiliaires comprend des graduations auxiliaires régulièrement espacées s'étendant à partir d'une origine coïncidant avec l'extrémité distale du cathéter.

5. Instrument chirurgical selon la revendication 4, caractérisé en ce que lesdites graduations auxiliaires sont espacées d'un centimètre entre elles.

6. Instrument chirurgical selon l'une des revendications 1 à 5, caractérisé en ce que les repères principaux et auxiliaires sont disposés sur le cathéter pour former deux échelles au voisinage l'une de l'autre, en vue de faciliter la lecture.

7. Instrument chirurgical selon l'une des revendications 1 à 6, caractérisé en ce que les repères principaux et auxiliaires sont de couleurs différentes.

## Claims

1. A surgical instrument comprising a needle (100) and an associated catheter (200) suitable for sliding inside a needle, in particular for performing epidural anesthesia, the needle being provided with graduations (130) that are axially distributed, and suitable for showing directly, merely by being read and without performing any calculations, the length of the needle extending from its distal end (1100) to each of said graduations (130), the instrument being characterized in that the needle is provided with a cursor (140) that is slidably mounted on the needle (100), that is suitable for bearing against the edge of an incision via which the needle is inserted, and that is designed to move along the needle through a distance corresponding to the length of needle inserted into said incision, and in that the catheter is provided with a set of main axial marks (210) suitable, when the catheter (200) is inserted in the needle (100), for coinciding with an index mark (I) defined on the needle, to show merely by being read and without performing any calculations, the length (L) of catheter extending beyond the distal end (1100) of the needle, and is provided with a set of auxiliary marks (220) easily distinguished from the main marks (210) and suitable for showing, merely by being read and without performing any calculations, the length of the catheter that extends from its distal end (2100) to each of said auxiliary marks (220).

2. A surgical instrument according to claim 1, characterized in that said set of main axial marks (210) comprises regularly spaced-apart main graduations extending from an origin (211) situated at a distance (D) from the distal end (2100) of the catheter, where said distance (D) corresponds to the distance between the distal end of the needle (1100) and the index (I) defined thereon.

3. A surgical instrument according to claim 2, characterized in that said main graduations (210) are spaced apart at one centimeter intervals.

4. A surgical instrument according to any one of claims 1 to 3, characterized in that said set of auxiliary marks comprises regularly spaced-apart auxiliary graduations extending from an origin that coincides with the distal end of the catheter.

5. A surgical instrument according to claim 4, characterized in that said auxiliary graduations are spaced apart at one centimeter intervals.

6. A surgical instrument according to any one of claims 1 to 5, characterized in that the main and auxiliary marks are disposed on the catheter to form two scales in the vicinity of each other, in order to facilitate reading thereof.

7. A surgical instrument according to any one of claims 1 to 6, characterized in that the main and auxiliary marks are of different colors.

**Patentansprüche**

1. Chirurgisches Instrument mit einer Nadel (100) und einem zugehörigen Katheter (200), welcher in der Nadel gleiten kann, insbesondere für Operationen mit Periduralanästhesie, wobei die Nadel mit axial verteilten Stricheinteilungen (130) versehen ist, die dafür geeignet sind, direkt durch einfaches Ablesen, ohne Berechnungen, die sich von dem entferntliegenden Ende (1100) bis zu jeder der Stricheinteilungen (130) erstreckende Nadellänge anzuzeigen, dadurch gekennzeichnet, daß die Nadel mit einem auf der Nadel (100) gleitend angeordneten Gleitschieber (140) versehen ist, welcher geeignet ist, sich in Auflage auf einer Kante eines Einschnittes anzuordnen, durch den die Nadel eingeführt wird, und welcher dafür eingerichtet ist, sich auf dieser um eine Strecke zu verschieben, die der in den Einschnitt eingeführten Nadellänge entspricht, und daß der Katheter mit einer Anordnung von axialen Hauptmarkierungen (210) versehen ist, welche, wenn der Katheter (200) in die Nadel (100) eingeführt ist, mit einem auf dieser definierten Index (I) übereinstimmen können, um durch einfaches Ablesen, ohne Berechnungen, die Katheterlänge (L) anzuzeigen, welche sich aus dem entfentliegenden Ende (1100) der Nadel heraus erstreckt, und er mit einer Anordnung von axialen Hilfsmarkierungen (220) versehen ist, welche sich leicht von den Hauptmarkierungen (210) unterscheiden lassen, und welche dafür geeignet sind, durch einfaches Ablesen, ohne Berechnungen, die Katheterlänge anzuzeigen, welche sich von seinem entfentliegenden Ende (2100) bis zu jeder der Hilfsmarkierungen (220) erstreckt.

2. Chirurgisches Instrument gemäß Anspruch 1, dadurch gekennzeichnet, daß die Anordnung von axialen Hauptmarkierungen (210) mit regelmäßigem Abstand angeordnete Hauptstricheinteilungen aufweist, die sich ausgehend von einem Ursprung (211) erstrecken, welcher mit einem Abstand (D) von dem entferntliegenden Ende (2100) des Katheters entsprechend dem Abstand zwischen dem entferntliegenden Ende der Nadel (1100) und dem auf dieser definierten Index (I) angeordnet ist.

3. Chirurgisches Instrument gemäß Anspruch 2, dadurch gekennzeichnet, daß die Hauptstricheinteilungen (210) mit einem Zentimeter voneinander beabstandet sind.

4. Chirurgisches Instrument gemäß einem der Ansprüche 1 bis 3, dadurch gekennzeichnet, daß die Anordnung von Hilfsmarkierungen mit regelmäßigem Abstand angeordnete Hilfsstricheinteilungen aufweist, die sich ausgebend von einem Ursprung erstrecken, welcher mit dem entferntliegenden Ende des Katheters übereinstimmt.

5. Chirurgisches Instrument gemäß Anspruch 4, dadurch gekennzeichnet, daß die Hilfsstricheinteilungen mit einem Zentimeter voneinander beabstandet sind.

6. Chirurgisches Instrument gemäß einem der Ansprüche 1 bis 5, dadurch gekennzeichnet, daß die Haupt- und Hilfsmarkierungen auf dem Katheter angeordnet sind, um zwei Skalen in Nachbarschaft zueinander zu bilden, um das Ablesen zu erleichtern.

7. Chirurgisches Instrument gemäß einem der Ansprüche 1 bis 6, dadurch gekennzeichnet, daß die Haupt- und Hilfsmarkierungen unterschiedliche Farben aufweisen.

## FIG.1

ETAT DE LA TECHNIQUE

## FIG.2

ETAT DE LA TECHNIQUE

## FIG.3

ETAT DE LA TECHNIQUE

FIG.4

FIG.5

FIG.6

8

FIG_7

FIG_8

FIG_9

FIG_10

9

FIG.11

FIG.12